# EUROPÄISCHE PATENTSCHRIFT

(11) **EP 4 404 886 B1**
(45) Veröffentlichungstag und Bekanntmachung des Hinweises auf die Patenterteilung: **26.08.2026**
(21) Anmeldenummer: 22786018.6
(22) Anmeldetag: 16.09.2022
(51) Int. Cl.: A61F 9/007

(54) **VERFAHREN ZUM BETREIBEN EINES OPHTHALMOCHIRURGISCHEN SYSTEMS UND OPHTHALMOCHIRURGISCHES SYSTEM**
METHOD FOR OPERATING AN OPHTHALMIC SURGICAL SYSTEM, AND OPHTHALMIC SURGICAL SYSTEM
PROCÉDÉ POUR FAIRE FONCTIONNER UN SYSTÈME DE CHIRURGIE OPHTALMOLOGIQUE ET SYSTÈME DE CHIRURGIE OPHTALMOLOGIQUE

(30) Priorität: 21.09.2021 DE 102021124405
(43) Veröffentlichungstag der Anmeldung: 31.07.2024
(73) Patentinhaber: Carl Zeiss Meditec AG, 07745 Jena (DE)
(72) Erfinder: KIBBEL, Steffen, 73466 Lauchheim (DE); KUEBLER, Christoph, 73447 Oberkochen (DE); KOHLHAMMER, Susanne, 89134 Blaustein (DE)
(74) Vertreter: Hofstetter, Schurack & Partner
(86) Internationale Anmeldenummer: PCT/EP2022/075853
(87) Internationale Veröffentlichungsnummer: WO 2023/046600

(56) Entgegenhaltungen:
- WO-A1-2010/056972
- DE-A1- 102018 130 376
- US-A1- 2020 100 851

## Beschreibung

Die Erfindung betrifft Verfahren zum Betreiben eines ophthalmochirurgischen Systems, wobei wenigstens zwei erste Fluidleitungen zum fluidtechnischen Koppeln einer Konsole des ophthalmochirurgischen Systems mit wenigstens einem medizinischen Behandlungsinstrument des ophthalmochirurgischen Systems an die Konsole angeschlossen werden, wobei die wenigstens zwei ersten Fluidleitungen wenigstens eine erste Irrigationsleitung und wenigstens eine erste Aspirationsleitung aufweisen, behandlungsinstrumentseitige Enden der wenigstens zwei ersten Fluidleitungen miteinander fluidtechnisch gekoppelt werden, und ein Behandlungsfluid mittels einer konsolenseitigen Pumpvorrichtung von einer Behandlungsfluidquelle durch die wenigstens zwei ersten Fluidleitungen zu einem Sammelbehältnis des ophthalmochirurgischen Systems gefördert wird. Ferner betrifft die Erfindung ein ophthalmochirurgisches System zur Behandlung eines Auges, mit zumindest wenigstens einem medizinischen Behandlungsinstrument zum Behandeln des Auges, einer Konsole zum Steuern des wenigstens einen medizinischen Behandlungsinstruments zumindest in einem bestimmungsgemäßen Betrieb, wenigstens zwei erste Fluidleitungen zum fluidtechnischen Koppeln der Konsole mit dem wenigstens einen medizinischen Behandlungsinstrument, wobei die wenigstens zwei ersten Fluidleitungen an die Konsole und an das wenigstens eine medizinische Behandlungsinstrument anschließbar sind, wobei die wenigstens zwei ersten Fluidleitungen wenigstens eine erste Irrigationsleitung und wenigstens eine erste Aspirationsleitung aufweisen, wobei das ophthalmochirurgische System ausgebildet ist, behandlungsinstrumentseitige Enden der wenigstens zwei ersten Fluidleitungen im mit der Konsole verbundenen Zustand miteinander fluidtechnisch zu koppeln und ein Behandlungsfluid mittels einer konsolenseitigen Pumpvorrichtung von einer Behandlungsfluidquelle durch die wenigstens zwei ersten Fluidleitungen zu einem Sammelbehältnis des ophthalmochirurgischen Systems zu fördern.

Ophthalmochirurgische Systeme, Konsolen hierfür sowie medizinische Behandlungsinstrumente, insbesondere Handstücke, und Verfahren hierfür sind im Stand der Technik bekannt, sodass es diesbezüglich eines gesonderten druckschriftlichen Nachweises dem Grunde nach nicht bedarf. Zum Beispiel zur Behandlung einer Augenlinsentrübung, in der Medizin auch als grauer Star bezeichnet, sind unterschiedliche chirurgische Techniken bekannt. Am weitesten verbreitet ist die Phakoemulsifikation, bei der eine dünne Hohlnadel eines Handstücks in einen Kapselsack, in dem die Augenlinse angeordnet ist, eingeführt und zu Ultraschallschwingungen angeregt wird. Mittels der vibrierenden Hohlnadel kann die Linse emulsifiziert werden, wobei hierbei freigesetzte Linsenpartikel über eine Aspirationsleitung mittels einer Pumpe abgesaugt werden können. Dabei wird ein Spülfluid beziehungsweise Behandlungsfluid, auch Irrigationsfluid genannt, zugeführt. Die Linsenpartikel werden zusammen mit diesem Fluid als Aspirationsfluid abgesaugt. Sobald die Linse vollständig emulsifiziert und entfernt ist, kann in den dann geleerten Kapselsack eine neue künstliche Linse eingesetzt werden. Hierdurch kann für den behandelten Patienten wieder ein gutes Sehvermögen erreicht werden.

Als Stand der Technik wird das Dokument DE102018130376 genannt. Das Dokument offenbart den Gegenstand des Oberbegiffs.

Ein fortschrittliches ophthalmochirurgisches System, welches sich für die Phakoemulsifikation als besonders geeignet herausgestellt hat, offenbart zum Beispiel die DE 10 2016 201 297 B3. Bei diesem System werden jeweils zwei strömungstechnisch parallelgeschaltete Fluidpumpen einer Pumpvorrichtung für die Irrigation sowie auch für die Aspiration genutzt. Jede der Fuidpumpen weist eine Pumpkammer und eine mittels eines bewegbar angeordneten Trennelements von der Pumpkammer getrennte Antriebskammer auf. Das Trennelement kann zum Beispiel elastisch ausgebildet und fest eingespannt sein oder auch verlagerbar zwischen den Kammern angeordnet sein. Die Antriebskammer wird für den bestimmungsgemäßen Betrieb der Fluidpumpe mit einem Antriebsfluid beaufschlagt, dessen Antriebsdruck zur Ausführung eines jeweiligen Pumphubs variiert wird. Dadurch verändert sich abhängig hiervon eine Auslenkungsposition des Trennelements, die sich entsprechend auf die Pumpkammer auswirkt. Die Pumpkammer ist mit dem jeweiligen Behandlungsfluid, beispielsweise dem Irrigationsfluid, dem Aspirationsfluid oder dergleichen, beaufschlagt. Durch geeignetes Steuern eines jeweiligen Einlass- und eines jeweiligen Auslassventils der Fluidpumpe, kann dann die Förderwirkung erreicht werden.

Eine Auslenkungsposition des Trennelements wird mittels eines der jeweiligen Fluidpumpe zugeordneten Auslenkungspositionssensors erfasst. Eine Steuereinrichtung des ophthalmochirurgischen Systems, insbesondere der Konsole, steuert die Funktion der Fluidpumpe zumindest abhängig von einem Sensorsignal des Auslenkungspositionssensors und einem mittels eines Antriebsdrucksensors bereitgestellten Antriebsdrucksignal. Die Steuereinrichtung kann ergänzend beispielsweise das Einlass- und das Auslassventil entsprechend steuern.

Durch wechselseitiges Betätigen der jeweils zwei parallelgeschalteten Fluidpumpen kann während einer Operation ein Volumenstrom mit sehr geringen Schwankungen erreicht werden. Dadurch kann sich ein nahezu konstanter Augeninnendruck im Kapselsack einstellen. Solange ausreichend Irrigationsfluid zugeführt werden kann, kann das System auch während einer sehr lang andauernden Operation nahezu ohne Unterbrechung des Irrigationsfluidstroms betrieben werden.

Häufig ist vorgesehen, dass ausgehend von einer Nichtgebrauchssituation die wenigstens zwei ersten Fluidleitungen für eine Ingebrauchsetzung des ophthalmochirurgischen Systems vor Durchführung einer ophthalmochirurgischen Behandlung mit dem Behandlungsfluid beaufschlagt werden. Vorzugsweise sollen die Fluidleitungen im Wesentlichen vollständig mit dem Behandlungsfluid beaufschlagt sein. Hierfür werden die Fluidleitungen an die Konsole angeschlossen und behandlungsinstrumentseitige Enden der wenigstens zwei ersten Fluidleitungen fluidtechnisch gekoppelt. Sodann wird mit der in der Regel konsolenseitigen Pumpvorrichtung das Behandlungsfluid durch die wenigstens zwei ersten Fluidleitungen gefördert, sodass diese, vorzugsweise vollständig, mit dem Behandlungsfluid gefüllt beziehungsweise gespült sind. Die fluidtechnische Kopplung der behandlungsinstrumentseitigen Enden wird dann aufgehoben und die Enden werden insbesondere mit dem medizinischen Behandlungsinstrument gekoppelt, sodass die medizinische Behandlung gestartet werden kann.

Soll das medizinische Behandlungsinstrument während der medizinischen Behandlung gewechselt werden oder ein weiteres medizinisches Behandlungsinstrument in Gebrauch gesetzt werden, ist die vorgenannte Inbetriebsetzung in der Regel manuell erneut durchzuführen. Dies ist während einer medizinischen Behandlung - wie einer Operation am Auge - zeitaufwendig und unter Umständen hinderlich.

Es ist die Aufgabe der Erfindung, ein Verfahren zum Betreiben eines ophthalmochirurgischen Systems sowie ein ophthalmochirurgisches System in Bezug auf ein Inbetriebsetzen von medizinischen Behandlungsinstrumenten zu verbessern.

Als Lösung werden mit der Erfindung ein Verfahren sowie ein ophthalmochirurgisches System zur Behandlung eines Auges gemäß den unabhängigen Ansprüchen vorgeschlagen.

Vorteilhafte Weiterbildungen ergeben sich durch Merkmale der abhängigen Ansprüche.

In Bezug auf ein gattungsgemäßes Verfahren wird mit der Erfindung insbesondere vorgeschlagen, dass konsolenseitig wenigstens eine zweite Fluidleitung für das Behandlungsfluid zum fluidtechnischen Koppeln der Konsole mit dem wenigstens einen medizinischen Behandlungsinstrument oder einem weiteren medizinischen Behandlungsinstrument angeschlossen wird, ein behandlungsinstrumentseitiges Ende der wenigstens einen zweiten Fluidleitung mit wenigstens einem der behandlungsinstrumentseitigen Enden der wenigstens zwei ersten Fluidleitungen fluidtechnisch gekoppelt wird, und das Behandlungsfluid mittels der konsolenseitigen Pumpvorrichtung durch die wenigstens eine zweite Fluidleitung gefördert wird.

In Bezug auf ein gattungsgemäßes ophthalmochirurgisches System wird mit der Erfindung insbesondere vorgeschlagen, dass das ophthalmochirurgische System wenigstens eine zweite Fluidleitung für das Behandlungsfluid aufweist, die zum fluidtechnischen Koppeln der Konsole mit dem wenigstens einen medizinischen Behandlungsinstrument oder einem weiteren medizinischen Behandlungsinstrument vorgesehen ist, wobei das ophthalmochirurgische System ausgebildet ist, ein behandlungsinstrumentseitiges Ende der wenigstens einen zweiten Fluidleitung mit wenigstens einem der behandlungsinstrumentseitigen Enden der wenigstens zwei ersten Fluidleitungen fluidtechnisch zu koppeln und das Behandlungsfluid mittels der konsolenseitigen Pumpvorrichtung durch die wenigstens eine zweite Fluidleitung zu fördern.

Die Erfindung basiert unter anderem auf dem Gedanken, den Nutzer des ophthalmochirurgischen Systems während der bestimmungsgemäßen Nutzung, beispielsweise bei einer medizinischen Behandlung am Auge, besser zu unterstützen. Insbesondere wenn während der bestimmungsgemäßen Nutzung beziehungsweise einer Operation am Auge das medizinische Behandlungsinstrument gewechselt werden soll oder ein weiteres medizinisches Behandlungsinstrument zum Einsatz gebracht werden soll, kann mit der Erfindung eine deutliche Unterstützung des Nutzers beziehungsweise Operateurs erreicht werden. Wenn beispielsweise der zusätzliche Einsatz einer zweiten Fluidleitung erforderlich wird, beispielsweise bei einer Inbetriebnahme eines weiteren medizinischen Behandlungsinstruments, ist es erforderlich, auch die wenigstens eine zweite Fluidleitung mit dem Behandlungsfluid zu beaufschlagen, bevor das weitere medizinische Behandlungsinstrument zum Einsatz gebracht werden kann. Handelt es sich bei dem Behandlungsfluid um eine Behandlungsflüssigkeit, beispielsweise eine Irrigationsflüssigkeit oder dergleichen, sollen die Fluidleitungen vorzugsweise keine Gasbeziehungsweise Luftblasen enthalten. Dadurch kann bereits bei Beginn der Nutzung des medizinischen Behandlungsinstruments eine vollständige zuverlässige Funktionalität realisiert werden. Ein derartiger Vorgang wird auch Priming genannt.

Während einer Operation erweist es sich als nachteilig für den Nutzer beziehungsweise Operateur, wenn das Priming den Operationsvorgang unterbricht, zumal das Priming bei einer ophthalmochirurgischen Operation für die Inbetriebnahme eines Handstücks mehrere Sekunden bis etwa 1,5 Minuten dauern kann. Die Erfindung ermöglicht es, dies zu vermeiden.

Das medizinische Behandlungsinstrument kann zum Beispiel ein Handstück für eine ophthalmochirurgische Behandlung sein, bei der eine Augenlinse eines Auges entfernt werden soll. Darüber hinaus kann es sich aber auch um ein Handstück handeln, welches für eine Hinterabschnittsoperation am Auge geeignet ist, ein Vitrektomie-Cutter oder dergleichen. Derartige medizinische Behandlungsinstrumente sind in der Regel über mindestens eine Aspirationsleitung beziehungsweise mindestens eine Irrigationsleitung, die in der Regel als Schlauch ausgebildet sind, mit der Konsole verbunden.

Diese Leitungen sowie auch mit dem Behandlungsfluid beaufschlagte Elemente beziehungsweise Einheiten der Konsole werden zumindest vor der bestimmungsgemäßen Nutzung und/oder gegebenenfalls auch während der Durchführung einer Operation gespült. Dies kann im Rahmen der erfindungsgemäßen Verfahrensführung erreicht werden. Dabei ist in der Regel vorgesehen, dass behandlungsinstrumentseitige Enden der wenigstens zwei ersten Fluidleitungen miteinander fluidtechnisch gekoppelt werden. Dies kann durch geeignetes Verbinden der behandlungsinstrumentseitigen Enden der ersten Fluidleitungen erfolgen, indem diese unmittelbar oder mittels eines fluidtechnischen Kopplungselements gekoppelt werden. Dem Grunde nach kann dies unter Umständen auch durch das medizinische Behandlungsinstrument selbst realisiert werden, indem das medizinische Behandlungsinstrument dafür ausgebildet ist, eine entsprechende, vorzugsweise steuerbare, fluidtechnische Verbindung bereitzustellen, beispielsweise indem eine entsprechende Kopplungsfunktion des medizinischen Behandlungsinstruments aktiviert wird oder dergleichen.

Zu diesem Zweck sowie auch zum Zwecke der bestimmungsgemäßen Nutzung weist das ophthalmochirurgische System eine Behandlungsfluidquelle auf, die beispielsweise konsolenseitig an einer entsprechenden Halterung angeordnet sein kann. Die Behandlungsfluidquelle kann das Behandlungsfluid bereitstellen, das beispielsweise das Irrigationsfluid, insbesondere eine Irrigationsflüssigkeit, sein kann. Durch die Irrigationsleitung wird das Behandlungsfluid von der Konsole zum behandlungsinstrumentseitigen Ende der Irrigationsleitung gefördert. Durch die fluidtechnische Kopplung mit der Aspirationsleitung am behandlungsinstrumentseitigen Ende wird sodann das Behandlungsfluid durch die Aspirationsleitung wieder zur Konsole gefördert und von dort einem Sammelbehältnis zugeführt. Das Sammelbehältnis kann beispielsweise ein Aufnahmebehälter sein, in dem das Behandlungsfluid konsolenseitig von der Aspirationsleitung gefördert und gespeichert wird.

Erfindungsgemäß ist konsolenseitig vorgesehen, dass wenigstens eine zweite Fluidleitung für das Behandlungsfluid zum fluidtechnischen Koppeln der Konsole mit dem wenigstens einen medizinischen Behandlungsinstrument oder einem weiteren medizinischen Behandlungsinstrument angeschlossen wird. Diese Fluidleitung kann beispielsweise eine Irrigationsleitung oder auch eine Aspirationsleitung sein. Darüber hinaus können natürlich auch mehrere Fluidleitungen vorgesehen sein, die je nach Bedarf Irrigationsleitungen und/oder Aspirationsleitungen sein können. Um den gewünschten Spülvorgang realisieren zu können, ist auch für die wenigstens eine zweite Fluidleitung vorgesehen, dass deren behandlungsinstrumentseitiges Ende mit wenigstens einem der behandlungsinstrumentseitigen Enden der wenigstens zwei ersten Fluidleitungen fluidtechnisch gekoppelt wird. Dadurch besteht die Möglichkeit, auch durch die wenigstens eine zweite Fluidleitung Behandlungsfluid zu fördern. Entsprechend ist die konsolenseitige Pumpvorrichtung ausgelegt, die zweite Fluidleitung mit dem Behandlungsfluid zu beaufschlagen.

Die mit dem Behandlungsfluid beaufschlagten Teile beziehungsweise Elemente beziehungsweise Einheiten der Konsole sind häufig in einem separaten, auswechselbaren Teil, welches in der Regel als Kassette ausgebildet ist, zusammengefasst angeordnet. Dadurch braucht die Konsole selbst nicht unmittelbar mit dem Behandlungsfluid in Kontakt zu kommen. Die Kassette ist ein auswechselbares Teil, welches in der Regel als Einwegteil, insbesondere als Wegwerfteil, ausgebildet ist. Dies ermöglicht es, auf einfache Weise eine gute Stabilität für die bestimmungsgemäße Nutzung erreichen zu können. So umfasst die Kassette unter anderem auch Elemente der Pumpvorrichtung, die unmittelbar mit dem Behandlungsfluid in Kontakt kommen. Die Pumpvorrichtung umfasst zum Beispiel für die Irrigation sowie auch für die Aspiration jeweils zumindest eine Fluidpumpe, die vorzugsweise von der Kassette vollständig erfasst ist. Ein Antrieb für die Fluidpumpe kann beispielsweise konsolenseitig vorgesehen sein, sodass möglichst nur Elemente durch die Kassette erfasst sind, die mit dem Behandlungsfluid unmittelbar in Kontakt kommen.

Darüber hinaus kann die Kassette auch Ventilelemente aufweisen, mit denen die Funktionen in Bezug auf das Fördern des Behandlungsfluids sowie auch das Führen des Behandlungsfluids zu den jeweiligen Fluidleitungen gesteuert werden kann. Auch hier kann vorgesehen sein, dass entsprechende Ventilantriebe konsolenseitig vorgesehen sind. Dadurch kann mit der Kassette ein Austauschteil beziehungsweise Einwegteil bereitgestellt werden, welches dazu dienen kann, die Sterilität des ophthalmochirurgischen Systems für eine jeweilige Operation am Auge, insbesondere in Bezug auf das Behandlungsfluid, sicherstellen zu können. Das Behandlungsfluid braucht nämlich nur durch die Kassette geführt zu werden, sodass das Behandlungsfluid die Konsole selbst nicht zu durchströmen und auch nicht mit der Konsole in Kontakt zu kommen braucht. Dadurch kann nach einer jeweiligen Nutzung des ophthalmochirurgischen Systems durch Austauschen der Kassette in der Konsole die Sterilität des ophthalmochirurgischen Systems jederzeit auf einfache Weise wiederhergestellt werden. Die Kassette kann ein Kassettengehäuse aufweisen, welches aus einem geeigneten Werkstoff, beispielsweise einem Kunststoff oder dergleichen, gebildet sein kann. Die Fluidpumpen können zumindest teilweise einstückig mit dem Kassettengehäuse ausgebildet sein.

Darüber hinaus wird ein Anschließen der wenigstens einen zweiten Fluidleitung detektiert, und die wenigstens eine zweite Fluidleitung wird mit dem Anschließen an die Konsole automatisiert mit dem Behandlungsfluid beaufschlagt. Dadurch kann erreicht werden, dass keine weiteren Aktionen vom Nutzer des ophthalmochirurgischen Systems durchgeführt zu werden brauchen, um die wenigstens eine zweite Fluidleitung zu spülen beziehungsweise zu primen.

Es wird ferner vorgeschlagen, dass die Fluidleitungen, insbesondere für eine Ingebrauchsetzung des ophthalmochirurgischen Systems, in einem Verfahrensschritt mit dem Behandlungsfluid beaufschlagt werden. Vorzugsweise werden die Fluidleitungen im Wesentlichen etwa zeitgleich mit dem Behandlungsfluid beaufschlagt, sodass sich am Ende dieses Verfahrensschritts in den Fluidleitungen vorzugsweise ausschließlich das Behandlungsfluid befindet.

Ferner wird vorgeschlagen, dass das Beaufschlagen der wenigstens einen zweiten Fluidleitung mit dem Behandlungsfluid während eines bestimmungsgemäßen Gebrauchs des wenigstens einen medizinischen Behandlungsinstruments erfolgt. Dadurch kann die wenigstens eine zweite Fluidleitung auch während der bestimmungsgemäßen Nutzung, beispielsweise dem Durchführen einer Operation am Auge, gespült werden. Dies ist besonders vorteilhaft, wenn während der Durchführung der Operation am Auge ein weiteres medizinisches Behandlungsinstrument zum Einsatz kommt, welches mit der wenigstens einen zweiten Fluidleitung fluidtechnisch gekoppelt ist. Der Nutzer des ophthalmochirurgischen Systems braucht daher seine Behandlung beziehungsweise Durchführung der Operation am Auge im Wesentlichen nicht zu unterbrechen.

Gemäß einer Weiterbildung wird vorgeschlagen, dass die wenigstens eine zweite Fluidleitung eine zweite Aspirationsleitung zum fluidtechnischen Koppeln des weiteren medizinischen Behandlungsinstruments ist. Darüber hinaus kann jedoch auch eine zweite Irrigationsleitung für das weitere medizinische Behandlungsinstrument vorgesehen sein.

Die diesbezüglichen Überlegungen sind entsprechend anwendbar. Beispielsweise kann es sich bei dem weiteren medizinischen Behandlungsinstrument um ein weiteres Handstück handeln. Durch die wenigstens eine zweite Aspirationsleitung kann das weitere medizinische Behandlungsinstrument beispielsweise gleichzeitig mit dem ersten medizinischen Behandlungsinstrument am gleichen Auge zum Einsatz kommen. In einem solchen Fall braucht eine zweite Irrigationsleitung für das weitere medizinische Behandlungsinstrument dem Grunde nach nicht vorgesehen zu sein. Besonders vorteilhaft können die wenigstens eine erste Aspirationsleitung und die wenigstens eine zweite Aspirationsleitung mit der gleichen Fluidpumpe der Pumpvorrichtung fluidtechnisch gekoppelt sein.

Es wird weiterhin vorgeschlagen, dass die Konsole die Fluidleitungen mittels einer Ventilvorrichtung individuell aktiviert. Dadurch kann bedarfsweise die jeweilige Fluidleitung für das jeweilige medizinische Behandlungsinstrument aktiviert beziehungsweise deaktiviert werden. Die Ventilvorrichtung kann hierfür ein oder mehrere geeignete Ventile umfassen, die konsolenseitig von der Steuereinrichtung gesteuert werden können.

Gemäß einer Weiterbildung wird vorgeschlagen, dass für wenigstens eine der Fluidleitungen ein strömungstechnischer Widerstand in Bezug auf das Fördern des Behandlungsfluids durch diese Fluidleitung ermittelt wird. Der strömungstechnische Widerstand kann beispielsweise mittels konsolenseitig vorgesehener Sensoren erfasst werden. Zu diesem Zweck kann zum Beispiel auch die Funktion der jeweiligen Fluidpumpe berücksichtigt werden. Mit dem strömungstechnischen Widerstand kann eine Funktion des ophthalmochirurgischen Systems überwacht werden.

Darüber hinaus wird vorgeschlagen, dass wenigstens ein Betriebsparameter des ophthalmochirurgischen Systems abhängig vom ermittelten strömungstechnischen Widerstand eingestellt wird. Der Betriebsparameter kann zum Beispiel die Pumpfunktion der wenigstens einen Fluidpumpe der Pumpvorrichtung der Konsole, auch eine Ventileinstellung der Ventileinrichtung und/oder dergleichen betreffen. Beispielsweise können aber auch Warn- oder Betriebsmeldungen erzeugt werden, die konsolenseitig mittels der Steuereinrichtung ausgegeben werden können, beispielsweise im Rahmen einer Anzeige für den Nutzer, einer Audioausgabe für den Nutzer und/oder dergleichen.

Weiterhin wird vorgeschlagen, dass das an die jeweilige Fluidleitung angeschlossene medizinische Behandlungsinstrument zumindest abhängig vom ermittelten strömungstechnischen Widerstand bestimmt wird, wobei insbesondere abhängig vom bestimmten medizinischen Behandlungsinstrument wenigstens ein Betriebsparameter des bestimmten medizinischen Behandlungsinstruments eingestellt wird. Beispielsweise kann ermittelt werden, ob es sich bei dem medizinischen Behandlungsinstrument um einen Vitrektomie-Cutter, ein Ultraschall-Handstück oder dergleichen handelt. Anhand des ermittelten beziehungsweise bestimmten medizinischen Behandlungsinstruments können dann die Funktionen für den bestimmungsgemäßen Betrieb ermittelt und entsprechende Betriebsparameter konsolenseitig eingestellt werden, sodass nutzerseitig der Einstellaufwand reduziert, wenn nicht sogar vollständig vermieden, werden kann. Darüber hinaus kann die Zuverlässigkeit verbessert werden, weil eine automatische Erkennung des medizinischen Behandlungsinstruments ermöglicht ist.

Die für das erfindungsgemäße Verfahren angegebenen Vorteile und Wirkungen gelten gleichermaßen auch für das ophthalmochirurgische System und umgekehrt. Insbesondere können daher Verfahrensmerkmale auch als Vorrichtungsmerkmale formuliert sein und umgekehrt.

Weitere Merkmale der Erfindung ergeben sich aus den Ansprüchen, den Figuren und der Figurenbeschreibung. Die vorstehend in der Beschreibung genannten Merkmale und Merkmalskombinationen, sowie die nachfolgend in der Figurenbeschreibung genannten und/oder in den Figuren alleine gezeigten Merkmale und Merkmalskombinationen sind nicht nur in der jeweils angegebenen Kombination, sondern auch in anderen Kombinationen verwendbar, ohne den Rahmen der Erfindung zu verlassen. Es sind somit auch Ausführungen von der Erfindung als umfasst und offenbart anzusehen, die in den Fig. nicht explizit gezeigt und erläutert sind, jedoch durch separierte Merkmalskombinationen aus den erläuterten Ausführungen hervorgehen und erzeugbar sind.

Die Erfindung wird durch die Ansprüche definiert.

Weitere Vorteile und Merkmale der Erfindung werden mit Bezug auf die nachfolgenden Zeichnungen erklärt, in welchen zeigen:
- Fig. 1: eine schematische Darstellung eines Ausführungsbeispiels eines ophthalmochirurgischen Systems mit einem Ausführungsbeispiel für eine ophthalmochirurgische Vorrichtung, die ein an einem Steuergerät der ophthalmochirurgischen Vorrichtung angeschlossenes ophthalmochirurgisches Handstück umfasst,
- Fig. 2: eine schematische Draufsicht auf eine Vorderseite einer Konsole des ophthalmochirurgischen Systems gemäß Fig. 1, bei der eine Kassette in einem Kassettenaufnahmebereich der Konsole angeordnet ist,
- Fig. 3: eine schematische Seitenansicht der Konsole gemäß Fig. 2, und
- Fig. 4: eine schematisch perspektivische Ansicht der Konsole gemäß Fig. 2.

In den Figuren bezeichnen gleiche Bezugszeichen gleiche Merkmale und Funktionen.

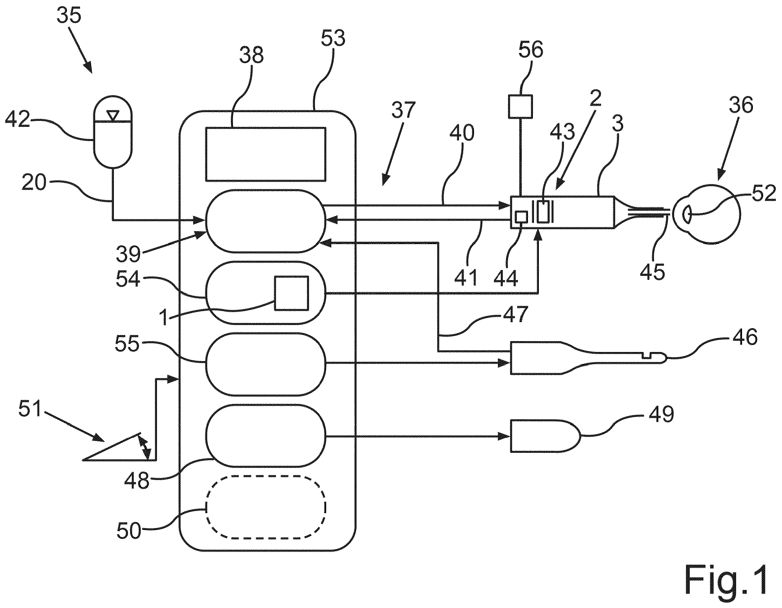

In Fig. 1 ist in schematischer Darstellung ein ophthalmisches mikrochirurgisches System beziehungsweise ein ophthalmochirurgisches System 35 zur Phako-Chirurgie an einem menschlichen Auge 36 gezeigt. Die Darstellung gemäß Fig. 1 zeigt symbolisch einige Komponenten des Systems 35 für die vereinfachte Erläuterung der prinzipiellen allgemeinen Funktionsweise des Systems 35.

Das ophthalmochirurgische System 35 weist eine Konsole 53 als Geräteeinheit auf, welche beispielsweise ein Rollwagen oder dergleichen sein kann. In oder an der Konsole 53 ist vorzugsweise eine Bedieneinheit 38 angeordnet. Diese Bedieneinheit 38 kann beispielsweise eine Benutzerschnittstelle, eine Eingabeeinheit, wie eine Tastatur oder dergleichen, und eine Anzeigeeinheit, wie einen Monitor oder ein Display, umfassen. Des Weiteren ist in der Konsole 53 vorzugsweise ein Fluidiksystem 39 angeordnet, welches eine Pumpvorrichtung und eine Steuereinrichtung zum Steuern der Pumpe und angeschlossener Komponenten aufweist. Das Fluidiksystem 39 weist eine Irrigationsvorrichtung mit einer Irrigationsleitung 40 und eine Aspirationsvorrichtung mit einer Aspirationsleitung 41 auf. Die Irrigationsvorrichtung weist einen Behälter 42 für Spülflüssigkeit, beispielsweise eine BSS-Lösung, welche ein Fluid für Irrigation ist, auf, welche zu einem Phako-Handstück geleitet wird. Das Phako-Handstück ist ein ophthalmochirurgisches Handstück 3. Es ist insbesondere Bestandteil des ophthalmochirurgischen Systems 35. Die Aspirationsleitung 41 ist ebenfalls mit dem ophthalmochirurgischen Handstück 3 verbunden.

Des Weiteren weist die Konsole 53 insbesondere eine Ultraschalleinheit 54 auf, welche zur Oszillationsanregung von piezoelektrischen Elementen 43 in dem ophthalmochirurgischen Handstück 3 ausgebildet ist, durch welche eine Hohlnadel 45 als Bearbeitungsnadel des ophthalmochirurgischen Handstücks 3 zur Oszillation angeregt wird. Die piezoelektrischen Elemente 43 bilden eine piezo-basierte Antriebseinheit 2. Die Ultraschalleinheit 54 umfasst vorliegend zumindest eine Steuereinheit 1 mit zumindest einem Wechselspannungsgenerator, einer Generatorsteuereinheit sowie einem Spannungssensor (nicht dargestellt).

Die Konsole 53 weist ferner insbesondere eine Steuereinheit 55 auf. Die Steuereinheit 55 kann auch zur Steuerung eines Vitrektomie-Handstücks 46, welches insbesondere Bestandteil des ophthalmochirurgischen Systems 35 sein kann, ausgebildet sein. Das Vitrektomie-Handstück 46 ist vorzugsweise auch mit dem Fluidiksystem 39, insbesondere durch eine Aspirationsleitung 47, verbunden. Darüber hinaus kann eine weitere Instrumentensteuereinheit 48 vorgesehen sein, welche ein vorzugsweise weiteres vorhandenes chirurgisches Instrument 49, beispielsweise für die Diathermie, steuert. Darüber hinaus kann das System 35 und insbesondere die Konsole 53 weitere Module und Steuereinheiten sowie Systeme umfassen, welche symbolisch durch die Einheit 50 dargestellt sind. Dadurch sind auch weitere interne Einheiten und auch Peripheriegeräte umfasst. Das ophthalmochirurgische System 35 weist des Weiteren vorzugsweise ein Fußschaltpult 51 auf, welches mit der Konsole 53, insbesondere mit Kommunikationseinrichtungen und Steuereinheiten der Konsole 53, verbunden ist.

In Fig. 1 ist darüber hinaus schematisch eine natürliche Augenlinse 52 im Auge 36 gezeigt. Diese Augenlinse 52 kann durch das ophthalmochirurgische System 35, insbesondere durch Phakoemulsifikation, entfernt werden.

In einer alternativen Ausführung kann vorgesehen sein, dass das ophthalmochirurgische System 35 einen zum Behälter 42 separaten Tank 44 (Fig. 1) aufweist. Bei einer derartigen Ausführung ist separat zur Irrigationsvorrichtung und separat zum Behälter 42 das Kühlfluid, welches zum Kühlen der Inzision vorgesehen ist, bereitgestellt. Der Tank 44 kann separat zum Handstück 3 angeordnet sein und beispielsweise über eine Leitung, wie eine Schlauchverbindung, mit dem Handstück 3 verbunden sein.

In einer weiteren Ausführung kann vorgesehen sein, dass der separate Tank 44 in dem Handstück 3 angeordnet ist. Er kann zerstörungsfrei unlösbar oder zerstörungsfrei lösbar in dem Handstück 3 angeordnet sein. Es kann auch vorgesehen sein, dass in dem Handstück 3 ein erster separater Tank 44 angeordnet ist und extern zum Handstück 3 ein weiterer separater Tank 56 angeordnet ist. Dieser zum Handstück 3 externe weitere separate Tank 56 kann mit dem ersten separaten Tank 44, der in dem Handstück 3 angeordnet ist, fluidleitend verbunden sein.

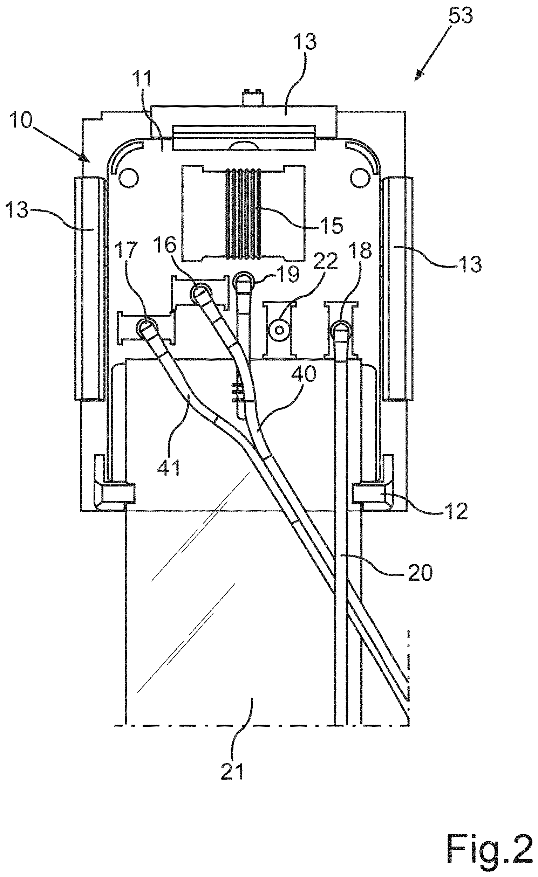

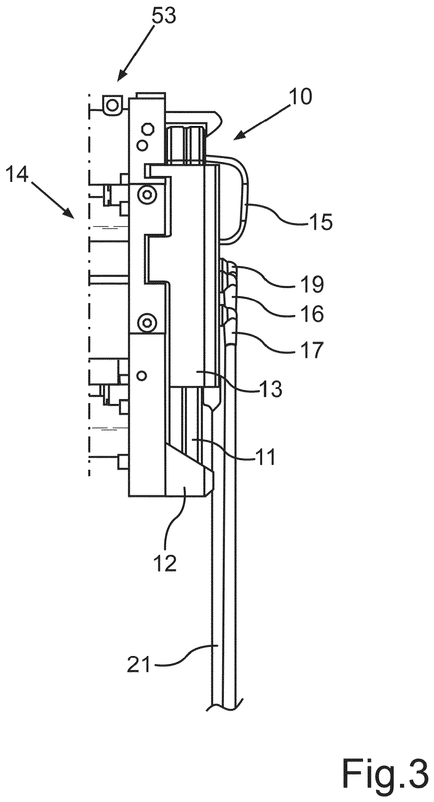

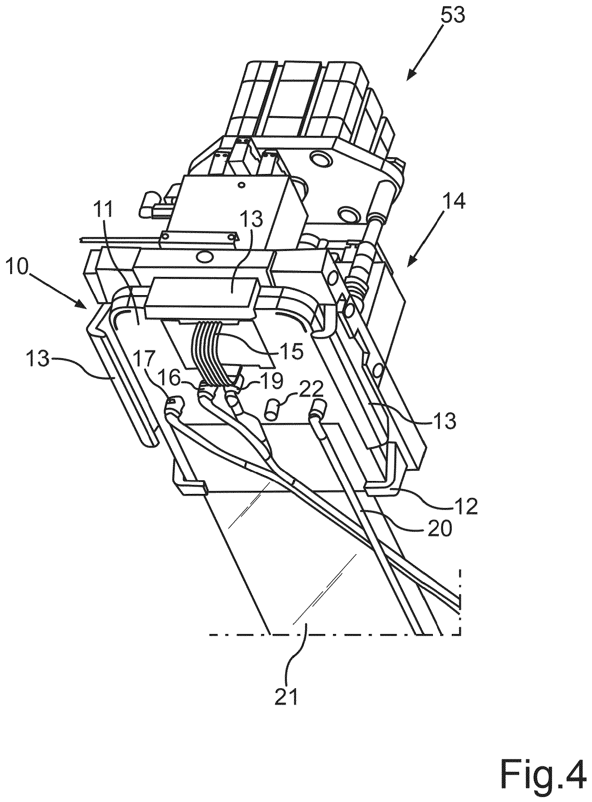

Die Fig. 2 bis 4 zeigen die Konsole 53 in unterschiedlichen Ansichten, sodass die Einzelheiten besser ersichtlich sind. Fig. 2 zeigt die Konsole 53 in einer Draufsicht auf eine Vorderseite, wohingegen Fig. 3 die Konsole 53 in einer schematischen Seitenansicht und Fig. 4 die Konsole 53 in einer schematischen perspektivischen Ansicht zeigt.

Aus Fig. 2 ist ersichtlich, dass die Konsole einen Kassettenaufnahmebereich 10 aufweist, in welchem eine Kassette 11 angeordnet ist. Der Kassettenaufnahmebereich 10 ist in einem unteren Bereich durch eine Kassettenabstützung 12 begrenzt, die die Kassette 11 in vertikaler Richtung aufgrund der Gewichtskraft hält. An Seiten des Kassettenaufnahmebereichs 10 sowie einer oberen Seite weist die Konsole 53 Halteklammern 13 auf, die die Kassette 11 im in den Kassettenaufnahmebereich 10 eingesetzten Zustand, wie er in den Fig. 2 bis 4 dargestellt ist, übergreifen und damit in dieser Position an der Konsole 53 fixieren.

Die Kassette 11 weist vorliegend sämtliche mit dem Irrigationsfluid und dem Aspirationsfluid in Kontakt stehenden Elemente der Konsole 53 auf. Die Kassette 11 ist als auswechselbares Einwegteil konzipiert, um auf diese Weise mit wenig Aufwand die Konsole 53 hinsichtlich der Sterilität für eine folgende Behandlung wiederherstellen zu können.

Zu diesem Zweck weist die Kassette 11 eine Pumpvorrichtung mit Fluidpumpen auf, die in den Figuren jedoch nicht dargestellt sind. Ebenso weist die Kassette 11 nicht dargestellte

Ventile einer Ventilvorrichtung auf, mit denen das Fördern des Behandlungsfluids, des Irrigationsfluids und des Aspirationsfluids, gesteuert werden kann. Zu diesem Zweck sind der Kassette 11 gegenüberliegend im Kassettenaufnahmebereich 10 konsolenseitige Steuer- und Betätigungselemente 14 vorgesehen, die im eingesetzten Zustand der Kassette 11 im Kassettenaufnahmebereich 10 die Kassette 11 kontaktieren, um die bestimmungsgemäße Funktionalität mittels der Kassette 11 hinsichtlich des Fluidiksystems 39 herstellen zu können.

Vorliegend ist vorgesehen, dass die Kassette 11 manuell in den Kassettenaufnahmebereich 10 eingesetzt und auch aus diesem nach Gebrauch wieder entfernt wird. Zu diesem Zweck weist die Kassette 11 eine Handhabe 15 auf.

Die Kassette 11 weist ferner einen Irrigationsanschluss 16 und einen Aspirationsanschluss 17 auf, die mit der jeweiligen Irrigationsleitung 40 und der Aspirationsleitung 41 fluidtechnisch verbunden sind. Die Irrigationsleitung 40 und die Aspirationsleitung 41 sind an das ophthalmochirurgische Handstück 3 angeschlossen. Darüber hinaus weist die Kassette 11 einen Irrigationsanschluss 18 auf, der über eine Irrigationsleitung 20 mit dem Behälter 42 fluidtechnisch gekoppelt ist. Über die Irrigationsleitung 20 wird die Irrigationsflüssigkeit aus dem Behälter 42 der Kassette 11 zugeführt. Ferner weist die Kassette 11 einen Aspirationsanschluss 19 auf, an dem ein Sammelbehältnis 21 angeschlossen ist. Das vom Handstück 3 über die Aspirationsleitung 41 abgesaugte Aspirationsfluid beziehungsweise die Aspirationsflüssigkeit wird über den Aspirationsanschluss 19 zum Sammelbehältnis 21 geführt und dort gesammelt beziehungsweise gespeichert. Das Sammelbehältnis 21 kann austauschbar sein.

Die Kassette 11 weist zumindest teilweise die Pumpvorrichtung und die Ventilvorrichtung auf, die durch die konsolenseitigen Steuer- und Betätigungselemente 14 erfasst sind, um die Fluidströme durch die Fluidleitungen entsprechend fördern zu können. So wird mittels der Kassette 11 das Irrigationsfluid, welches über den Irrigationsanschluss 18 der Kassette 11 zugeführt wird, über den Irrigationsanschluss 16 und die Irrigationsleitung 40 dem Handstück 3 zugeführt. Umgekehrt wird Aspirationsfluid, welches vom Handstück 3 aufgenommen wird, über die Aspirationsleitung 41 und dem Aspirationsanschluss 17 der Kassette 11 und von dort über den Aspirationsanschluss 19 dem Sammelbehältnis 21 zugeführt. Durch die Pumpvorrichtung in Verbindung mit der Ventilvorrichtung wird die gewünschte Förderwirkung erreicht.

In den Fig. 2 bis 4 ist ferner dargestellt, dass die Kassette 11 einen zweiten Aspirationsanschluss 22 aufweist. Dieser dient, wie aus Fig. 1 ersichtlich ist, zum Anschließen der zweiten Aspirationsleitung 47 des Vitrektomie-Handstücks 46. Im bestimmungsgemäßen Betrieb wird mittels der Kassette 11 Aspirationsfluid somit nicht nur über das Handstück 3, sondern ebenfalls über das Vitrektomie-Handstück 46 abgeführt.

Zum Betreiben des ophthalmochirurgischen Systems 35 ist vorgesehen, dass handstückseitige Enden der Aspirationsleitung 41 und der Irrigationsleitung 40, die vorliegend erste Fluidleitungen bilden, miteinander strömungstechnisch gekoppelt werden. Mittels der Pumpvorrichtung der Kassette 11 wird das Irrigationsfluid vom Behälter 42 durch die Irrigationsleitung 40 und die Aspirationsleitung 41 zum Sammelbehältnis 21 des ophthalmochirurgischen Systems 35 gefördert.

Weiterhin wird konsolenseitig die zweite Aspirationsleitung 47 mit dem Vitrektomie-Handstück 46 an die Kassette 11 der Konsole 53 angeschlossen, die mit dem Vitrektomie-Handstück 46 fluidtechnisch gekoppelt ist. Ein Vitrektomie-handstückseitiges Ende der Aspirationsleitung 47 wird mit dem handstückseitigen Ende der Irrigationsleitung 40 fluidtechnisch gekoppelt. Mittels der konsolenseitigen Pumpvorrichtung wird über geeignetes Steuern der Ventilvorrichtung das Behandlungsfluid beziehungsweise Irrigationsfluid durch die zweite Aspirationsleitung 47 gefördert. Dadurch können sämtliche Fluidleitungen 40, 41, 47 gespült werden beziehungsweise geprimt werden. Dies erfolgt vorzugsweise in einem einzigen Verfahrensschritt, sodass für die Durchführung der Operation kein weiterer Spülvorgang mehr vorgenommen zu werden braucht.

Vorliegend ist vorgesehen, dass die Konsole 53 über die Kassette 11, insbesondere dort vorgesehene Sensoren, das Anschließen der zweiten Aspirationsleitung 47 detektiert. Daraufhin wird die zweite Aspirationsleitung 47 automatisiert mit dem Irrigationsfluid beaufschlagt. Es braucht vom Nutzer daher keine separate Handlung mehr vorgenommen zu werden.

Es kann alternativ oder ergänzend vorgesehen sein, dass die zweite Aspirationsleitung 47 erst dann mit dem Irrigationsfluid beaufschlagt wird, wenn das Vitrektomie-Handstück 46 während einer Operation in Betrieb genommen wird, bei der das Handstück 3 sich bereits in der Nutzung befindet. Zu diesem Zweck kann vorgesehen sein, dass mittels der Ventilvorrichtung der Konsole 53 in Verbindung mit der Kassette 11 eine individuelle Steuerung realisiert werden kann.

Darüber hinaus ist vorliegend vorgesehen, dass für die Fluidleitungen 40, 41, 47 mittels zumindest teilweise konsolenseitig, insbesondere kassettenseitig, vorgesehener Sensoren jeweilige strömungstechnische Widerstände in Bezug auf das Fördern des Irrigationsfluids beziehungsweise des Aspirationsfluids ermittelt werden. Abhängig hiervon bestimmt die Steuereinheit 55 beziehungsweise die Ultraschalleinheit 54 das jeweils angeschlossene medizinische Behandlungsinstrument, hier das Handstück 3 beziehungsweise das Vitrektomie-Handstück 46. Abhängig hiervon werden Betriebsparameter für die Handstücke 3, 46 automatisiert eingestellt. Vom Nutzer des ophthalmochirurgischen System 35 brauchen daher diesbezüglich keine separaten Einstellungen vorgenommen zu werden. Dadurch kann die Zuverlässigkeit und die Sicherheit weiter verbessert werden.

Insbesondere ist es auf diese Weise möglich, ein jeweiliges Handstück 3, 46 zu identifizieren. Alternativ oder ergänzend kann natürlich auch vorgesehen sein, dass das Handstück über RFID oder dergleichen identifiziert werden kann. Die erforderlichen Betriebsparameter können in einer Speichereinheit der Konsole 53 abrufbar gespeichert sein. Es kann aber auch vorgesehen sein, dass die Konsole 53 über eine Kommunikationsverbindung zu einer zentralen Datenbank die entsprechenden Betriebsparameter für das jeweils identifizierte Handstück 3, 46 abruft und die entsprechenden Einstellungen vornimmt.

Die Erfindung ermöglicht es also, Handstücke vorzugsweise vor einer Operation zu spülen beziehungsweise zu primen. Darüber hinaus besteht natürlich die Möglichkeit, ein während einer Operation neu hinzugenommenes Handstück individuell zu spülen beziehungsweise zu primen. Dabei sollte jedoch bedacht werden, dass das Primen die Operation unter Umständen unterbrechen kann, weil für das bereits im Betrieb befindliche Handstück die fluidtechnische Versorgung während des Primens beziehungsweise Spülens gegebenenfalls nicht aufrechterhalten werden kann.

Vorzugsweise umfasst das Spülen beziehungsweise Primen auch die Kassette 11 der Konsole 53.

Darüber hinaus kann natürlich vorgesehen sein, dass, wenn mehr als ein einziges Handstück an der Konsole 53 beziehungsweise Kassette 11 angeschlossen ist, die angeschlossenen Handstücke durch geeignetes Steuern nacheinander gespült beziehungsweise geprimt werden, ohne dass der Nutzer weitere Aktionen durchzuführen braucht.

Mit dem Betriebsparameter kann vorliegend zum Beispiel ein fluidtechnischer Durchmesser gemeint sein, der zum Beispiel in einem Bereich von etwa 19 Gauge bis etwa 27 Gauge betragen kann, das heißt, in einem Bereich für einen Innendurchmesser von etwa 1,1 Millimeter bis etwa 0,4 Millimeter. Der fluidtechnische Widerstand ist vorzugsweise in Form von Daten tabellarisch hinterlegt. Abhängig vom ermittelten fluidtechnischen Widerstand kann auf dem Innendurchmesser der Fluidleitungen 40, 41, 47 zurückgerechnet werden, und es können die Betriebsparameter darauf eingestellt werden.

Insgesamt ermöglicht es die Erfindung, die Nutzung des ophthalmochirurgischen Systems weiter zu verbessern.

Die Ausführungsbeispiele dienen ausschließlich der Erläuterung der Erfindung und sind für diese nicht beschränkend.

### Bezugszeichenliste

- 1: Steuereinheit
- 2: Antriebseinheit
- 3: Handstück
- 10: Kassettenaufnahmebereich
- 11: Kassette
- 12: Kassettenabstützung
- 13: Halteklammern
- 14: Betätigungselemente
- 15: Handhabe
- 16: Irrigationsanschluss
- 17: Aspirationsanschluss
- 18: Irrigationsanschluss
- 19: Aspirationsanschluss
- 20: Irrigationsleitung
- 21: Sammelbehältnis
- 22: Aspirationsanschluss
- 35: ophthalmochirurgisches System
- 38: Bedieneinheit
- 39: Fluidiksystems
- 40: Fluidleitungen
- 40: Irrigationsleitung
- 41: Aspirationsleitung
- 42: Behälter
- 46: Vitrektomie-Handstück
- 47: Aspirationsleitung
- 53: Konsole
- 54: Ultraschalleinheit
- 55: Steuereinheit

## Patentansprüche

1. Verfahren zum Betreiben eines ophthalmochirurgischen Systems (35), wobei:
- wenigstens zwei erste Fluidleitungen (40, 41) zum fluidtechnischen Koppeln einer Konsole (53) des ophthalmochirurgischen Systems (35) mit wenigstens einem medizinischen Behandlungsinstrument (3) des ophthalmochirurgischen Systems (35) an die Konsole (53) angeschlossen werden, wobei die wenigstens zwei ersten Fluidleitungen (40, 41) wenigstens eine erste Irrigationsleitung (40) und wenigstens eine erste Aspirationsleitung (41) aufweisen,
- behandlungsinstrumentseitige Enden der wenigstens zwei ersten Fluidleitungen (40, 41) miteinander fluidtechnisch gekoppelt werden, um die wenigstens zwei ersten Fluidleitungen (40, 41) zu primen,
- ein Behandlungsfluid mittels einer konsolenseitigen Pumpvorrichtung von einer Behandlungsfluidquelle (42) durch die wenigstens zwei ersten Fluidleitungen (40, 41) zu einem Sammelbehältnis (21) des ophthalmochirurgischen Systems (35) gefördert wird,
- konsolenseitig wenigstens eine zweite Fluidleitung (47) für das Behandlungsfluid zum fluidtechnischen Koppeln der Konsole (53) mit dem wenigstens einen medizinischen Behandlungsinstrument oder einem weiteren medizinischen Behandlungsinstrument (46) angeschlossen wird,
- ein behandlungsinstrumentseitiges Ende der wenigstens einen zweiten Fluidleitung (47) mit wenigstens einem der behandlungsinstrumentseitigen Enden der wenigstens zwei ersten Fluidleitungen (40, 41) fluidtechnisch gekoppelt wird, und
- das Behandlungsfluid mittels der konsolenseitigen Pumpvorrichtung durch die wenigstens eine zweite Fluidleitung (47) gefördert wird, um die wenigstens eine zweite Fluidleitung (47) zu primen,
**dadurch gekennzeichnet, dass**
ein Anschließen der wenigstens einen zweiten Fluidleitung (47) detektiert und die wenigstens eine zweite Fluidleitung (47) mit dem Anschließen an die Konsole (53) automatisiert mit dem Behandlungsfluid beaufschlagt wird.

2. Verfahren nach Anspruch 1,
**dadurch gekennzeichnet, dass**
die Fluidleitungen (40, 41), insbesondere für eine Ingebrauchsetzung des ophthalmochirurgischen Systems (35), in einem Verfahrensschritt mit dem Behandlungsfluid beaufschlagt werden.

3. Verfahren nach einem der vorhergehenden Ansprüche,
**dadurch gekennzeichnet, dass**
das Beaufschlagen der wenigstens einen zweiten Fluidleitung (47) mit dem Behandlungsfluid während eines bestimmungsgemäßen Gebrauchs des wenigstens einen medizinischen Behandlungsinstruments (3) erfolgt.

4. Verfahren nach einem der vorhergehenden Ansprüche,
**dadurch gekennzeichnet, dass**
die wenigstens eine zweite Fluidleitung (47) eine zweite Aspirationsleitung zum fluidtechnischen Koppeln des weiteren medizinischen Behandlungsinstruments (46) ist.

5. Verfahren nach einem der vorhergehenden Ansprüche,
**dadurch gekennzeichnet, dass**
die Konsole (53) die Fluidleitungen (40, 41, 47) mittels einer Ventilvorrichtung individuell aktiviert.

6. Verfahren nach einem der vorhergehenden Ansprüche,
**dadurch gekennzeichnet, dass**
für wenigstens eine der Fluidleitungen (40, 41, 47) ein strömungstechnischer Widerstand in Bezug auf das Fördern des Behandlungsfluids durch diese Fluidleitung (40, 41, 47) ermittelt wird.

7. Verfahren nach Anspruch 6,
**dadurch gekennzeichnet, dass**
wenigstens ein Betriebsparameter des ophthalmochirurgischen Systems (35) abhängig vom ermittelten strömungstechnischen Widerstand eingestellt wird.

8. Verfahren nach Anspruch 6 oder 7,
**dadurch gekennzeichnet, dass**
das an die jeweilige Fluidleitung (40, 41, 47) angeschlossene medizinische Behandlungsinstrument (3, 46) zumindest abhängig vom ermittelten strömungstechnischen Widerstand bestimmt wird, wobei insbesondere abhängig vom bestimmten medizinischen Behandlungsinstrument (3, 46) wenigstens ein Betriebsparameter des bestimmten medizinischen Behandlungsinstruments (3, 46) eingestellt wird.

9. Ophthalmochirurgisches System (35) zur Behandlung eines Auges (6), mit zumindest:
- wenigstens einem medizinischen Behandlungsinstrument (3) zum Behandeln des Auges (6),
- einer Konsole (53) zum Steuern des wenigstens einen medizinischen Behandlungsinstruments (3),
- wenigstens zwei erste Fluidleitungen (40, 41) zum fluidtechnischen Koppeln der Konsole (53) mit dem wenigstens einen medizinischen Behandlungsinstrument (3), wobei die wenigstens zwei ersten Fluidleitungen (40, 41) an die Konsole (53) und an das wenigstens eine medizinische Behandlungsinstrument (3) anschließbar sind, wobei die wenigstens zwei ersten Fluidleitungen (40, 41) wenigstens eine erste Irrigationsleitung (40) und wenigstens eine erste Aspirationsleitung (41) aufweisen,
- wobei das ophthalmochirurgische System (35) ausgebildet ist, behandlungsinstrumentseitige Enden der wenigstens zwei ersten Fluidleitungen (40, 41) im mit der Konsole (53) verbundenen Zustand miteinander fluidtechnisch zu koppeln und ein Behandlungsfluid mittels einer konsolenseitigen Pumpvorrichtung von einer Behandlungsfluidquelle (42) durch die wenigstens zwei ersten Fluidleitungen (40, 41) zu einem Sammelbehältnis (21) des ophthalmochirurgischen Systems (35) zu fördern,
- wenigstens eine zweite Fluidleitung (47) für das Behandlungsfluid, die zum fluidtechnischen Koppeln der Konsole (53) mit dem wenigstens einen medizinischen Behandlungsinstrument oder einem weiteren medizinischen Behandlungsinstrument (46) ausgebildet ist,
- wobei das ophthalmochirurgische System (35) ausgebildet ist, ein behandlungsinstrumentseitiges Ende der wenigstens einen zweiten Fluidleitung (47) mit wenigstens einem der behandlungsinstrumentseitigen Enden der wenigstens zwei ersten Fluidleitungen (40, 41) fluidtechnisch zu koppeln und das Behandlungsfluid mittels der konsolenseitigen Pumpvorrichtung durch die wenigstens eine zweite Fluidleitung (47) zu fördern,
**dadurch gekennzeichnet, dass**
das ophthalmochirurgische System (35) ausgebildet ist, ein Anschließen der wenigstens einen zweiten Fluidleitung (47) zu detektieren und die wenigstens eine zweite Fluidleitung (47) mit dem Anschließen an die Konsole (53) automatisiert mit dem Behandlungsfluid zu beaufschlagen.

## Claims

1. Method for operating an ophthalmic surgical system (35), wherein:
- at least two first fluid lines (40, 41) for fluidically coupling a console (53) of the ophthalmic surgical system (35) to at least one medical treatment instrument (3) of the ophthalmic surgical system (35) are connected to the console (53), with the at least two first fluid lines (40, 41) comprising at least one first irrigation line (40) and at least one first aspiration line (41),
- treatment instrument-side ends of the at least two first fluid lines (40, 41) are fluidically coupled to one another in order to prime the at least two first fluid lines (40, 41),
- a treatment fluid is conveyed through the at least two first fluid lines (40, 41) from a treatment fluid source (42) to a collecting container (21) of the ophthalmic surgical system (35) by means of a console-side pumping apparatus,
- at least one second fluid line (47) for the treatment fluid is connected on the console side for fluidically coupling the console (53) to the at least one medical treatment instrument or a further medical treatment instrument (46),
- a treatment instrument-side end of the at least one second fluid line (47) is fluidically coupled to at least one of the treatment instrument-side ends of the at least two first fluid lines (40, 41), and
- the treatment fluid is conveyed through the at least one second fluid line (47) by means of the console-side pumping apparatus in order to prime the at least one second fluid line (47),
**characterized in that**
a connection of the at least one second fluid line (47) is detected, and the treatment fluid is automatically applied to the at least one second fluid line (47) upon connection to the console (53).

2. Method according to Claim 1,
**characterized in that**
the treatment fluid is applied to the fluid lines (40, 41) in one method step, in particular for putting the ophthalmic surgical system (35) into use.

3. Method according to any of the preceding claims, **characterized in that**
the treatment fluid is applied to the at least one second fluid line (47) during an intended use of the at least one medical treatment instrument (3).

4. Method according to any of the preceding claims, **characterized in that**
the at least one second fluid line (47) is a second aspiration line for fluidically coupling the further medical treatment instrument (46).

5. Method according to any of the preceding claims, **characterized in that**
the console (53) activates the fluid lines (40, 41, 47) individually by means of a valve apparatus.

6. Method according to any of the preceding claims, **characterized in that**
for at least one of the fluid lines (40, 41, 47), a fluid resistance is ascertained in relation to the conveyance of the treatment fluid through this fluid line (40, 41, 47).

7. Method according to Claim 6,
**characterized in that**
at least one operational parameter of the ophthalmic surgical system (35) is set on the basis of the ascertained fluid resistance.

8. Method according to Claim 6 or 7,
**characterized in that**
the medical treatment instrument (3, 46) connected to the respective fluid line (40, 41, 47) is determined at least on the basis of the ascertained fluid resistance, with at least one operational parameter of the determined medical treatment instrument (3, 46) being set, in particular on the basis of the determined medical treatment instrument (3, 46).

9. Ophthalmic surgical system (35) for treating an eye (6), at least having:
- at least one medical treatment instrument (3) for treating the eye (6),
- a console (53) for controlling the at least one medical treatment instrument (3),
- at least two first fluid lines (40, 41) for fluidically coupling the console (53) to the at least one medical treatment instrument (3), with the at least two first fluid lines (40, 41) being connectable to the console (53) and to the at least one medical treatment instrument (3), with the at least two first fluid lines (40, 41) comprising at least one first irrigation line (40) and at least one first aspiration line (41),
- wherein the ophthalmic surgical system (35) is designed to fluidically couple treatment instrument-side ends of the at least two first fluid lines (40, 41) to one another in the console (53) connected state and convey a treatment fluid through the at least two first fluid lines (40, 41) from a treatment fluid source (42) to a collecting container (21) of the ophthalmic surgical system (35) by means of a console-side pumping apparatus,
- at least one second fluid line (47) for the treatment fluid and designed to fluidically couple the console (53) to the at least one medical treatment instrument or a further medical treatment instrument (46),
- wherein the ophthalmic surgical system (35) is designed to fluidically couple a treatment instrument-side end of the at least one second fluid line (47) to at least one of the treatment instrument-side ends of the at least two first fluid lines (40, 41) and convey the treatment fluid through the at least one second fluid line (47) by means of the console-side pumping apparatus,
**characterized in that**
the ophthalmic surgical system (35) is designed to detect a connection of the at least one second fluid line (47) and automatically apply the treatment fluid to the at least one second fluid line (47) upon connection to the console (53).

## Revendications

1. Procédé pour faire fonctionner un système de chirurgie ophtalmologique (35), dans lequel :
- au moins deux premières conduites de fluide (40, 41) sont raccordées à une console (53) pour accoupler fluidiquement ladite console (53) du système de chirurgie ophtalmologique (35) avec au moins un instrument de traitement médical (3) du système de chirurgie ophtalmologique (35), lesdites au moins deux premières conduites de fluide (40, 41) comportant au moins une première conduite d'irrigation (40) et au moins une première conduite d'aspiration (41),
- les extrémités côté instrument de traitement desdites au moins deux premières conduites de fluide (40, 41) sont accouplées fluidiquement l'une à l'autre afin d'amorcer lesdites au moins deux premières conduites de fluide (40, 41),
- un fluide de traitement est acheminé au moyen d'un dispositif de pompage côté console, d'une source de fluide de traitement (42) jusqu'à un récipient de collecte (21) du système de chirurgie ophtalmologique (35) à travers lesdites au moins deux premières conduites de fluide (40, 41),
- côté console, au moins une deuxième conduite de fluide (47) pour le fluide de traitement est raccordée pour accoupler fluidiquement la console (53) audit au moins un instrument de traitement médical ou à un autre instrument de traitement médical (46),
- une extrémité côté instrument de traitement de ladite au moins une deuxième conduite de fluide (47) est accouplée fluidiquement à au moins une des extrémités côté instrument de traitement desdites au moins deux premières conduites de fluide (40, 41), et
- le fluide de traitement est acheminé à travers ladite au moins une deuxième conduite de fluide (47) au moyen du dispositif de pompage côté console afin d'amorcer ladite au moins une deuxième conduite de fluide (47), **caractérisé en ce que**
un raccordement de ladite au moins une deuxième conduite de fluide (47) est détecté et ladite au moins une deuxième conduite de fluide (47) est automatiquement alimentée en fluide de traitement lors de son raccordement à la console (53).

2. Procédé selon la revendication 1,
**caractérisé en ce que**
les conduites de fluide (40, 41), en particulier pour une mise en service du système de chirurgie ophtalmologique (35), sont alimentées en fluide de traitement lors d'une étape du procédé.

3. Procédé selon l'une quelconque des revendications précédentes,
**caractérisé en ce que**
ladite au moins une deuxième conduite de fluide (47) est alimentée en fluide de traitement pendant une utilisation conforme à sa destination dudit au moins un instrument de traitement médical (3).

4. Procédé selon l'une quelconque des revendications précédentes,
**caractérisé en ce que**
ladite au moins une deuxième conduite de fluide (47) est une deuxième conduite d'aspiration permettant d'accoupler fluidiquement ledit autre instrument de traitement médical (46).

5. Procédé selon l'une quelconque des revendications précédentes,
**caractérisé en ce que**
la console (53) active individuellement les conduites de fluide (40, 41, 47) au moyen d'un dispositif à vannes.

6. Procédé selon l'une quelconque des revendications précédentes,
**caractérisé en ce que**
pour au moins l'une des conduites de fluide (40, 41, 47), une résistance à l'écoulement par rapport à l'acheminement du fluide de traitement à travers ladite conduite de fluide (40, 41, 47) est déterminée.

7. Procédé selon la revendication 6,
**caractérisé en ce que**
au moins un paramètre de fonctionnement du système de chirurgie ophtalmologique (35) est réglé en fonction de la résistance à l'écoulement déterminée.

8. Procédé selon la revendication 6 ou 7,
**caractérisé en ce que**
l'instrument de traitement médical (3, 46) raccordé à la conduite de fluide respective (40, 41, 47) est déterminé au moins en fonction de la résistance à l'écoulement déterminée, au moins un paramètre de fonctionnement de l'instrument de traitement médical (3, 46) déterminé étant notamment réglé en fonction de l'instrument de traitement médical (3, 46) déterminé.

9. Système de chirurgie ophtalmologique (35) pour le traitement d'un œil (6), comprenant au moins :
- au moins un instrument de traitement médical (3) pour le traitement de l'œil (6),
- une console (53) pour commander ledit au moins un instrument de traitement médical (3),
- au moins deux premières conduites de fluide (40, 41) pour accoupler fluidiquement la console (53) audit au moins un instrument de traitement médical (3), lesdites au moins deux premières conduites de fluide (40, 41) pouvant être raccordées à la console (53) et audit au moins un instrument de traitement médical (3), lesdites au moins deux premières conduites de fluide (40, 41) comportant au moins une première conduite d'irrigation (40) et au moins une première conduite d'aspiration (41),
- le système de chirurgie ophtalmologique (35) étant conçu pour accoupler fluidiquement l'une à l'autre des extrémités côté instrument de traitement desdites au moins deux premières conduites de fluide (40, 41) dans l'état raccordé à la console (53), et pour acheminer un fluide de traitement au moyen d'un dispositif de pompage côté console, d'une source de fluide de traitement (42) jusqu'à un récipient de collecte (21) du système de chirurgie ophtalmologique (35) à travers lesdites au moins deux premières conduites de fluide (40, 41),
- au moins une deuxième conduite de fluide (47) pour le fluide de traitement, laquelle est conçue pour accoupler fluidiquement la console (53) audit au moins un instrument de traitement médical ou à un autre instrument de traitement médical (46),
- le système de chirurgie ophtalmologique (35) étant conçu pour accoupler fluidiquement une extrémité côté instrument de traitement de ladite au moins une deuxième conduite de fluide (47) à au moins une des extrémités côté instrument de traitement desdites au moins deux premières conduites de fluide (40, 41) et pour acheminer le fluide de traitement à travers ladite au moins une deuxième conduite de fluide (47) au moyen du dispositif de pompage côté console,
**caractérisé en ce que**
le système de chirurgie ophtalmologique (35) est conçu pour détecter un raccordement de ladite au moins une deuxième conduite de fluide (47) et pour alimenter automatiquement en fluide de traitement ladite au moins une deuxième conduite de fluide (47) lors de son raccordement à la console (53).
